# EUROPÄISCHE PATENTSCHRIFT

(11) **EP 1 294 298 B1**
(45) Veröffentlichungstag und Bekanntmachung des Hinweises auf die Patenterteilung: **10.11.2004**
(21) Anmeldenummer: 00936585.9
(22) Anmeldetag: 26.06.2000
(51) Int. Cl.: A61B 17/80

(54) **KNOCHENPLATTE FÜR DIE OSTEOSYNTHESE**
BONE PLATE FOR OSTEOSYNTHESIS
PLAQUE OSSEUSE POUR OSTEOSYNTHESE

(43) Veröffentlichungstag der Anmeldung: 26.03.2003
(73) Patentinhaber: SYNTHES AG Chur, 7002 Chur (CH)
(72) Erfinder: FRIGG, Robert, CH-2544 Bettlach (CH); FERUS, Robert, CH-2544 Bettlach (CH)
(74) Vertreter: Lusuardi, Werther Giovanni, Dr.
(86) Internationale Anmeldenummer: PCT/CH2000/000346
(87) Internationale Veröffentlichungsnummer: WO 2002/000127

(56) Entgegenhaltungen:
- WO-A-97/09000
- WO-A-98/06345
- US-A- 5 002 544
- US-A- 5 474 553
- US-A- 5 733 287

## Beschreibung

Die Erfindung betrifft eine Knochenplatte für die Osteosynthese gemäss dem Oberbegriff des Patentanspruchs 1 sowie eine Fixationsvorrichtung gemäss dem Oberbegriff des Patentanspruchs 18.

Aus der WO 98/06345 und aus der US-A-5 002 544 sind jeweils Knochenplatten bekannt, bei welchen die Kontaktfläche mit dem Knochen reduziert ist.

Aus der WO97/09000 ist eine Knochenplatte bekannt, bei welcher über ein konisches Aussengewinde am Schraubenkopf und einem entsprechenden konischen Innengewinde im Plattenloch eine winkel- und axial stabile Schraubenverankerung erzielbar ist. Ein Nachteil dieser bekannten Knochenplatte besteht aber darin, dass eine minimale Plattendicke notwendig ist, um die Schraube mit ihrem Kopf ausreichend in der Platte zu fixieren. Bei Plattendicken unter 2 mm beginnt die erreichte winkel- und axiale Stabilität der Schraubenverankerung kritisch zu werden.

Hier will die Erfindung Abhilfe schaffen. Der Erfindung liegt das Problem zugrunde, eine Knochenplatte zu schaffen, welche auch bei einer relativ geringen Plattendicke eine sichere winkel- und axiale Stabilität der Schraubenverankerung ermöglicht.

Die Erfindung löst die gestellte Aufgabe mit einer Knochenplatte, welche die Merkmale des Anspruchs 1 aufweist.

Die Verdickung im Lochbereich der Knochenplatte - im Vergleich zum Zwischenlochbereich - erfolgt durch einen über die Unterseite der Platte hervorstehenden Wulst, vorzugsweise von hohlkegelförmiger Gestalt. Fabrikatorisch kann dies beispielsweise durch konisches Tiefziehen der Platte im Bereich um das Plattenloch herum erzielt werden.
Die Dicke der Knochenplatte im Bereich der Löcher mit Wulst sollte das 1,1 bis 4,0-fache, vorzugsweise, das 1,5 bis 2,0-fache der Dicke im Bereich zwischen diesen Löchern ausmachen. Der Wulst sollte dabei um 0,1 bis 3,0 mm, vorzugsweise um 0,5 bis 1,0 mm über die Unterseite hinausragen.
Die Dicke der Knochenplatte im Bereich zwischen den Löchern mit Wulst kann 1 bis 2 mm betragen, vorzugsweise 0,8 bis 1,2 mm.

Bei einer bevorzugten Ausführungsform, bei welcher der Wulst hohlkegelförmig ausgebildet ist, beträgt der äussere Konuswinkel 5° bis 120°, vorzugsweise 40° bis 100°.

Bei einer bevorzugten Ausführungsform sind die einen Wulst aufweisenden Löcher mit einem Innengewinde versehen. Das Innengewinde kann entweder kreiszylindrisch oder konisch ausgebildet sein; im letzteren Fall verjüngen sich vorzugsweise die einen Wulst aufweisenden Löcher ebenfalls zur Unterseite hin konisch.
Das sich konisch verjüngende Innengewinde kann einen Konuswinkel von 5° bis 50°, vorzugsweise von 10° bis 30° aufweisen.

Das Innengewinde kann entweder ein- oder zweigängig sein. Die Gewindesteigung für ein eingängiges Innengewinde sollte zwischen 0,4 und 1,5 mm, vorzugsweise zwischen 0,60 und 1,25 mm liegen. Für ein zweigängiges Innengewinde sollte die Gewindesteigung zwischen 0,2 und 1,5 mm, vorzugsweise zwischen 0,3 und 0,8 mm liegen.

Die Zentralachsen der Löcher mit Wulst sind im wesentlichen senkrecht zur Oberseite angeordnet und verlaufen vorzugsweise parallel zueinander.

Mit der erfindungsgemässen Knochenplatte werden vorteilhafterweise Knochenschrauben mit einem konischen Schraubenkopf verwendet, welche vorzugsweise ein Aussengewinde tragen. Der Konuswinkel des konischen Schraubenkopfes sollte dabei vorteilhafterweise dem Konuswinkel des allfällig konischen Innengewindes des Plattenloches entsprechen. Dank dieser Gestaltung wird die Knochenplatte rund um die Plattenlöcher herum verdickt. In dieser Verdickung (bzw. in diesem axial künstlich verlängerten Plattenloch) können mehr Gewindegänge geschnitten werden, als dies bei einer konstanten Plattendicke möglich wäre. Dies führt zu einer verbesserten Schraubenverankerung, ohne dass dabei die mechanischen Eigenschaften der Knochenplatte, d.h. ihre Flexibilität und Anpassbarkeit an den Knochen, grundsätzlich verändert würden.

Die erfindungsgemässe Knochenplatte lässt sich vorallem überall dort verwenden, wo dünne Knochenplatten von Vorteil sind, insbesondere im Wirbelsäulenbereich, am Becken und an Röhrenknochen.

Die Erfindung und Weiterbildungen der Erfindung werden im folgenden anhand der teilweise schematischen Darstellung eines Ausführungsbeispiels näher erläutert.

Es zeigt:

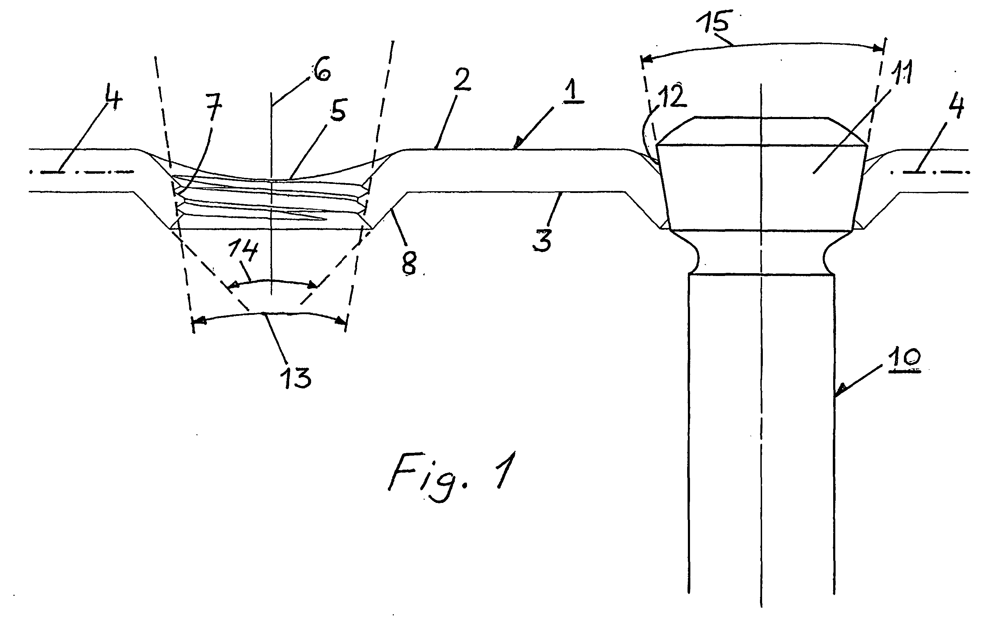

Fig. 1 einen Längsschnitt durch die erfindungsgemässe Knochenplatte mit einer darin eingeführten Knochenschraube.

Die in Fig. 1 dargestellte einstückige Knochenplatte 1 für die Osteosynthese besitzt eine Oberseite 2, eine knochenseitige Unterseite 3 sowie mehrere die Ober- mit der Unterseite 2;3 verbindende, entlang der Plattenlängsachse 4 angeordnete Löcher 5 mit Zentralachsen 6 für die Aufnahme von Knochenschrauben 10, wobei in der Figur lediglich zwei Löcher 5 dargestellt sind; weitere Löcher 5 können - je nach Länge der Knochenplatte 1 - links und rechts davon angebracht sein.
Die Zentralachsen 6 der Löcher 5 stehen senkrecht zur Oberseite 2 und verlaufen somit parallel zueinander.

Die Plattendicke im Zwischenlochbereich beträgt 1 mm. Die beiden in der Figur dargestellten Löcher 5 weisen einen über die Unterseite 3 ragenden, zur Zentralachse 6 konzentrischen, hohlkegelförmigen Wulst 8 auf.
Die Löcher 5 sind mit einem eingängigen Innengewinde 7 versehen, welches sich zur Unterseite 3 hin konisch verjüngt (es kann aber auch ein kreiszylindrisches und/oder ein zweigängiges Innengewinde verwendet werden). Der Konuswinkel 13 des Innengewindes 7 beträgt 20°. Der äussere Konuswinkel 14 des Wulstes 8 beträgt 90°. Die Gewindesteigung des Innengewindes 7 beträgt 0,8 mm (bei einem zweigängigen Innengewinde würde die Steigung entsprechend 0,4 mm betragen).

Durch den 0,8 mm hohen Wulst 8 weist die Knochenplatte im Bereich der Löcher 5 eine grössere Dicke auf als im Bereich zwischen diesen Löchern 5 (1 mm dick) und zwar um das 1,8-fache.

Die im rechten Loch 5 eingesetzte Knochenschraube 10 weist einen konischen Schraubenkopf 11 auf, der ein Aussengewinde 12 trägt. Der Konuswinkel 15 des konischen Schraubenkopfes 11 beträgt 20° und entspricht somit dem Konuswinkel des konischen Innengewindes 7.

Die Auflagefläche der Knochenplatte 1 ist auf die kreisringförmigen, knochenseitigen Frontpartien der Wülste 8 reduziert, was zu einer verbesserten Heilung der unter der Knochenplatte 1 zu liegenden Knochenpartie führt.

## Patentansprüche

1. Knochenplatte (1) für die Osteosynthese, mit einer Oberseite (2), einer knochenseitigen Unterseite (3) sowie mehreren die Ober- mit der Unterseite (2;3) verbindenden, entlang der Plattenlängsachse (4) angeordneten Löchern (5) mit Zentralachsen (6) für die Aufnahme von Knochenschrauben (10), wobei mindestens zwei der Löcher (5) einen über die Unterseite (3) ragenden, zur Zentralachse (6) konzentrischen Wulst (8) aufweisen,
**dadurch gekennzeichnet, dass**
die Knochenplatte (1) einstückig ausgebildet ist.

2. Knochenplatte (1) nach Anspruch 1, **dadurch gekennzeichnet, dass** die einen Wulst (8) aufweisenden Löcher (5) mit einem vorzugsweise kreiszylindrischen Innengewinde (7) versehen sind.

3. Knochenplatte (1) nach Anspruch 1 oder 2, **dadurch gekennzeichnet, dass** die einen Wulst (8) aufweisenden Löcher (5) sich zur Unterseite (3) hin konisch verjüngen.

4. Knochenplatte (1) nach Anspruch 2 oder 3, **dadurch gekennzeichnet, dass** sich das Innengewinde (7) zur Unterseite (3) hin konisch verjüngt.

5. Knochenplatte nach Anspruch 4, **dadurch gekennzeichnet, dass** das sich konisch verjüngende Innengewinde (7) einen Konuswinkel (13) von 5° bis 50°, vorzugsweise von 10° bis 30° aufweist.

6. Knochenplatte (1) nach einem der Ansprüche 2 bis 5, **dadurch gekennzeichnet, dass** das Innengewinde (7) ein eingängiges Gewinde ist.

7. Knochenplatte (1) nach Anspruch 6, **dadurch gekennzeichnet, dass** die Gewindesteigung des eingängigen Innengewindes (7) zwischen 0,4 und 1,5 mm, vorzugsweise zwischen 0,60 und 1,25 mm liegt.

8. Knochenplatte (1) nach einem der Ansprüche 1 bis 5, **dadurch gekennzeichnet, dass** das Innengewinde (7) ein zweigängiges Gewinde ist.

9. Knochenplatte (1) nach Anspruch 8, **dadurch gekennzeichnet, dass** die Gewindesteigung des zweigängigen Innengewindes (7) zwischen 0,2 und 1,5 mm, vorzugsweise zwischen 0,3 und 0,8 mm liegt.

10. Knochenplatte (1) nach einem der Ansprüche 1 bis 9, **dadurch gekennzeichnet, dass** sie im Bereich der Löcher (5) mit Wulst (8) eine grössere Dicke aufweist als im Bereich zwischen diesen Löchern (5).

11. Knochenplatte (1) nach Anspruch 10, **dadurch gekennzeichnet, dass** die Dicke der Knochenplatte im Bereich der Löcher (5) mit Wulst (8) das 1,1 bis 4,0-fache, vorzugsweise das 1,5 bis 2,0-fache der Dicke im Bereich zwischen diesen Löchern (5) ausmacht.

12. Knochenplatte (1) nach einem der Ansprüche 1 bis 11, **dadurch gekennzeichnet, dass** der Wulst (8) um 0,1 bis 3,0 mm, vorzugsweise um 0,5 bis 1,0 mm über die Unterseite (3) hinausragt.

13. Knochenplatte (1) nach einem der Ansprüche 1 bis 12, **dadurch gekennzeichnet, dass** sich der Wulst (8) von der Knochenplatte (1) weg hohlkegelförmig verjüngt.

14. Knochenplatte (1) nach Anspruch 13, **dadurch gekennzeichnet, dass** der hohlkegelförmige Wulst (8) einen äusseren Konuswinkel (14) von 5° bis 120°, vorzugsweise von 40° bis 100° aufweist.

15. Knochenplatte (1) nach einem der Ansprüche 1 bis 14, **dadurch gekennzeichnet, dass** die Dicke der Knochenplatte (1) im Bereich zwischen den Löchern (5) mit Wulst (8) 1 bis 2 mm beträgt.

16. Knochenplatte (1) nach einem der Ansprüche 1-14, **dadurch gekennzeichnet, dass** die Dicke der Knochenplatte (1) im Bereich zwischen den Löchern (5) mit Wulst (8) 0,8 bis 1,2 mm beträgt.

17. Knochenplatte (1) nach einem der Ansprüche 1 bis 16, **dadurch gekennzeichnet, dass** die Zentral achsen (6) der Löcher (5) mit Wulst (8) im wesentlichen senkrecht zur Oberseite (2) angeordnet sind und vorzugsweise parallel zueinander verlaufen.

18. Fixationsvorrichtung mit einer Knochenplatte (1) nach einem der Ansprüche 1 bis 17, **dadurch gekennzeichnet, dass** sie mindestens zwei Knochenschrauben (10) mit einem konischen Schraubenkopf (11) umfasst, der ein Aussengewinde (12) trägt.

19. Fixationsvorrichtung nach Anspruch 18, **dadurch gekennzeichnet, dass** der Konuswinkel (15) des konischen Schraubenkopfes (11) dem Konuswinkel (13) des konischen Innengewindes (7) entspricht.

## Claims

1. A bone plate (1) for osteosynthesis including a top surface (2), a bottom surface (3) facing the bone, and a plurality of holes (5) with central axes (6) for receiving bone screws (10), arranged along the longitudinal axis (4) of the plate and connecting the top surface (2) with the bottom surface (3), at least two of the holes (5) being provided with a bulge (8) arranged concentrically to the central axis (6) and projecting over the bottom surface (3),
**characterized in that**
the bone plate (1) consists of a single piece.

2. A bone plate (1) as claimed in claim 1, **characterised in that** the holes (5) provided with a bulge (8) have a preferably circularly cylindrical internal thread (7).

3. A bone plate (1) as claimed in claim 1 or 2, **characterised in that** the holes (5) provided with a bulge (8) taper conically to the bottom surface (3).

4. A bone plate (1) as claimed in claim 1 or 3, **characterised in that** the internal thread (7) tapers conically to the bottom surface (3).

5. A bone plate as claimed in claim 4, **characterised in that** the conically tapered internal thread (7) has a cone angle (13) of between 5 and 50 degrees, preferably between 10 and 30 degrees.

6. A bone plate (1) as claimed in any of the claims 2 to 5, **characterised in that** the internal thread (7) is a single thread.

7. A bone plate (1) as claimed in claim 6, **characterised in that** the thread pitch of the single internal thread (7) is between 0.4 and 1.5 mm, preferably between 0.60 and 1.25 mm.

8. A bone plate (1) as claimed in any of the claims 1 to 5, **characterised in that** the internal thread (7) is a double thread.

9. A bone plate (1) as claimed in claim 8, **characterised in that** the thread pitch of the double internal thread (7) is between 0.2 and 1.5 mm, preferably between 0.3 and 0.8 mm.

10. A bone plate (1) as claimed in any of the claims 1 to 9, **characterised in that** it has a greater thickness in the areas of the holes (5) provided with a bulge (8) than in the area between said holes (5).

11. A bone plate (1) as claimed in claim 10, **characterised in that** the thickness of the bone plate as measured in the areas of the holes (5) provided with a bulge (8) is between 1.1 and 4.0 times, preferably between 1.5 and 2.0 times thicker than in the area between said holes (5).

12. A bone plate (1) as claimed in any of the claims 1 to 11, **characterised in that** the bulge (8) projects over the bottom surface (3) by an amount between 0.1 and 3.0 mm, preferably between 0.5 and 1.0 mm.

13. A bone plate (1) as claimed in any of the claims 1 to 12, **characterised in that** the bulge (8), beginning from the bone plate (1), tapers in the form of a hollow cone.

14. A bone plate (1) as claimed in claim 13, **characterised in that** the bulge (8) shaped in the form of a hollow cone has an outside cone angle (14) of between 5 and 120 degrees, preferably between 40 and 100 degrees.

15. A bone plate (1) as claimed in any of the claims 1 to 14, **characterised in that** the thickness of the bone plate (1) as measured in the area between the holes (5) provided with a bulge (8) is between 1 and 2 mm.

16. A bone plate (1) as claimed in one of the claims 1 to 14, **characterised in that** the thickness of the bone plate (1) as measured in the area between the holes (5) provided with a bulge (8) is between 0.8 and 1.2 mm.

17. A bone plate (1) as claimed in any of the claims 1 to 16, **characterised in that** the central axes (6) of the holes (5) provided with a bulge (8) are substantially arranged vertically to the top surface (2) and preferably extend parallel to one another.

18. A fixation device including a bone plate (1) as claimed in any of the claims 1 to 17, **characterised in that** it comprises at least two bone screws (10) having a conical screw head (11) provided with an external thread (12).

19. A fixation device as claimed in claim 18, **characterised in that** the cone angle (15) of the conical screw head (11) corresponds to the cone angle (13) of the conical internal thread (7).

## Revendications

1. Plaque d'ostéosynthèse (1) comportant une face supérieure (2), une face inférieure (3) située du côté de l'os ainsi que plusieurs trous (5), avec des axes centraux respectifs (6), reliant la face supérieure (2) à la face inférieure (3), disposés le long de l'axe longitudinal (4) de la plaque et destinés à recevoir des vis d'ostéosynthèse (10), au moins deux des trous (5) présentant un renflement (8) concentrique à l'axe central (6) faisant saillie par rapport à la face inférieure (3),
**caractérisée en ce que**
la plaque d'ostéosynthèse (1) est formée en une seule pièce.

2. Plaque d'ostéosynthèse (1) selon la revendication 1, **caractérisée en ce que** les trous (5) présentant un renflement (8) sont pourvus d'un filet intérieur (7) de préférence de forme cylindrique circulaire.

3. Plaque d'ostéosynthèse (1) selon la revendication 1 ou 2, **caractérisée en ce que** les trous (5) présentant un renflement (8) vont en s'amincissant de manière conique vers la face inférieure (3).

4. Plaque d'ostéosynthèse (1) selon la revendication 2 ou 3, **caractérisée en ce que** le filet intérieur (7) va en s'amincissant de manière conique vers la face inférieure (3).

5. Plaque d'ostéosynthèse selon la revendication 4, **caractérisée en ce que** le filet intérieur (7) qui va en s'amincissant de manière conique présente un angle de cône (13) compris entre 5° et 50°, de préférence entre 10° et 30°.

6. Plaque d'ostéosynthèse (1) selon l'une des revendications 2 à 5, **caractérisée en ce que** le filet intérieur (7) est un filet simple.

7. Plaque d'ostéosynthèse (1) selon la revendication 6, **caractérisée en ce que** le pas de filetage du filet intérieur simple (7) est compris entre 0,4 et 1,5 mm, de préférence entre 0,60 et 1,25 mm.

8. Plaque d'ostéosynthèse (1) selon l'une des revendications 1 à 5, **caractérisée en ce que** le filet intérieur (7) est un filet double.

9. Plaque d'ostéosynthèse (1) selon la revendication 8, **caractérisée en ce que** le pas de filetage du filet intérieur double (7) est compris entre 0,2 et 1,5 mm, de préférence entre 0,3 et 0,8 mm.

10. Plaque d'ostéosynthèse (1) selon l'une des revendications 1 à 9, **caractérisée en ce que** celle-ci présente, dans les zones entourant respectivement les trous (5) avec renflement (8), une épaisseur supérieure à celle de la zone située entre lesdits trous (5).

11. Plaque d'ostéosynthèse (1) selon la revendication 10, **caractérisée en ce que** l'épaisseur de la plaque d'ostéosynthèse dans les zones entourant respectivement les trous (5) avec renflement (8) est supérieure entre 1,1 et 4,0 fois, de préférence entre 1,5 et 2,0 fois, à l'épaisseur de la zone située entre lesdits trous (5).

12. Plaque d'ostéosynthèse (1) selon l'une des revendications 1 à 11, **caractérisée en ce que** le renflement (8) fait saillie de 0,1 à 3,0 mm, de préférence de 0,5 à 1,0 mm, par rapport à la face inférieure (3).

13. Plaque d'ostéosynthèse (1) selon l'une des revendications 1 à 12, **caractérisée en ce que** le renflement (8) va en s'amincissant sous forme de cône creux depuis la plaque d'ostéosynthèse (1).

14. Plaque d'ostéosynthèse (1) selon la revendication 13, **caractérisée en ce que** le renflement (8) en forme de cône creux présente un angle de cône extérieur (14) compris entre 5° et 120°, de préférence entre 40° et 100°.

15. Plaque d'ostéosynthèse (1) selon l'une des revendications 1 à 14, **caractérisée en ce que** l'épaisseur de la plaque d'ostéosynthèse (1) est comprise, dans la zone située entre les trous (5) avec renflement (8), entre 1 et 2 mm.

16. Plaque d'ostéosynthèse (1) selon l'une des revendications 1 à 14, **caractérisée en ce que** l'épaisseur de la plaque d'ostéosynthèse (1) est comprise, dans la zone située entre les trous (5) avec renflement (8), entre 0,8 et 1,2 mm.

17. Plaque d'ostéosynthèse (1) selon l'une des revendications 1 à 16, **caractérisée en ce que** les axes centraux respectifs (6) des trous (5) avec renflement (8) sont disposés essentiellement de manière perpendiculaire à la face supérieure (2) et s'étendent, de préférence, parallèlement les uns aux autres.

18. Dispositif de fixation comportant une plaque d'ostéosynthèse (1) selon l'une des revendications 1 à 17, **caractérisé en ce que** celui-ci comprend au moins deux vis d'ostéosynthèse (10) avec une tête de vis (11) de forme conique pourvue d'un filet extérieur (12).

19. Dispositif de fixation selon la revendication 18, **caractérisé en ce que** l'angle de cône (15) de la tête de vis (11) de forme conique correspond à l'angle de cône (13) du filet intérieur (7) de forme conique.
